(19) Europäisches Patentamt
European Patent Office
Office européen des brevets

(11) **EP 3 509 650 B1**

(12) **EUROPEAN PATENT SPECIFICATION**

(45) Date of publication and mention
of the grant of the patent:
**04.11.2020 Bulletin 2020/45**

(51) Int Cl.:
*A61L 27/32* (2006.01)    *A61L 27/30* (2006.01)
*A61L 27/54* (2006.01)    *A61K 38/40* (2006.01)

(21) Application number: **17783996.6**

(22) Date of filing: **11.09.2017**

(86) International application number:
**PCT/IB2017/055464**

(87) International publication number:
**WO 2018/047130 (15.03.2018 Gazette 2018/11)**

(54) **IMPLANTABLE MEDICAL DEVICES HAVING A COATING LAYER WITH ANTIMICROBIAL PROPERTIES BASED ON NANOSTRUCTURED HYDROXYAPATITE**

IMPLANTIERBARE MEDIZINISCHE VORRICHTUNGEN MIT EINER BESCHICHTUNG MIT ANTIMIKROBIELLEN EIGENSCHAFTEN AUF DER BASIS VON NANOSTRUKTURIERTEM HYDROXYAPATIT

DISPOSITIFS MÉDICAUX IMPLANTABLES COMPORTANT UNE COUCHE DE REVÊTEMENT AYANT DES PROPRIÉTÉS ANTIMICROBIENNES À BASE D'HYDROXYAPATITE NANOSTRUCTURÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.09.2016 IT 201600091766**

(43) Date of publication of application:
**17.07.2019 Bulletin 2019/29**

(73) Proprietor: **Innovaplants S.r.l.**
**41126 Modena (IT)**

(72) Inventors:
• **ROVERI, Norberto**
**40133 Bologna (IT)**
• **LELLI, Marco**
**40063 Monghidoro (BO) (IT)**
• **MASETTI, Massimo**
**40012 Calderara Di Reno (BO) (IT)**
• **ZANASI, Stefano**
**41014 Castelvetro Di Modena (MO) (IT)**
• **VENTURA, Carlo**
**40122 Bologna (IT)**
• **GRUPPIONI, Andrea**
**40133 Bologna (IT)**

(74) Representative: **Bottero, Carlo et al**
**Barzanò & Zanardo Milano S.p.A.**
**Via Borgonuovo, 10**
**20121 Milano (IT)**

(56) References cited:
**CN-A- 103 952 745      CN-A- 104 415 401**
**US-A1- 2012 087 954**

• **MICHELE IAFISCO ET AL: "Adsorption and spectroscopic characterization of lactoferrin on hydroxyapatite nanocrystals", DALTON TRANSACTIONS, vol. 40, no. 4, 1 January 2011 (2011-01-01), page 820, XP055035146, ISSN: 1477-9226, DOI: 10.1039/C0DT00714E**
• **SUTHA S ET AL: "Enhancement of antimicrobial and long-term biostability of the zinc-incorporated hydroxyapatite coated 316L stainless steel implant for biomedical application", CERAMICS INTERNATIONAL, ELSEVIER, AMSTERDAM, NL, vol. 39, no. 5, 23 December 2012 (2012-12-23), pages 5205-5212, XP028526870, ISSN: 0272-8842, DOI: 10.1016/J.CERAMINT.2012.12.019**

- STANIC V ET AL: "Synthesis, characterization and antimicrobial activity of copper and zinc-doped hydroxyapatite nanopowders", APPLIED SURFACE SCIENCE, ELSEVIER, AMSTERDAM, NL, vol. 256, no. 20, 1 August 2010 (2010-08-01), pages 6083-6089, XP027076744, ISSN: 0169-4332, DOI: 10.1016/J.APSUSC.2010.03.124 [retrieved on 2010-06-04]

- DOS SANTOS E A ET AL: "Oriented hydroxyapatite single crystals produced by the electrodeposition method", MATERIALS SCIENCE AND ENGINEERING: B, ELSEVIER, AMSTERDAM, NL, vol. 169, no. 1-3, 25 May 2010 (2010-05-25), pages 138-144, XP027038227, ISSN: 0921-5107 [retrieved on 2009-10-28]

**Description**

The present invention relates to implantable medical devices that can be used in particular in the orthopaedic and traumatological field, having a coating layer with antimicrobial properties based on nanostructured hydroxyapatite.

Implantable medical devices are typically sterilized before use and are packed in packages that preserve the sterility of the device until the package is opened, and therefore at the moment of use.

According to the environment in which it is used, the device can be contaminated with microbes before use or during insertion, or after insertion or implantation if the implant site in the patient is contaminated, as a result, for example, of a trauma or faulty or inadequate sterile procedures. The microbial contamination of medical devices can cause serious infections in the patient, which frequently cannot be easily treated for a variety reasons, among which the formation of microbial biofilms that are highly resistant to antibiotic therapies.

It is also known that a joint prosthesis can give a reaction to a foreign body within the human body and can therefore be rejected by the same. Even if, in most cases, our body accepts joint prostheses, these can be colonized by microbes once implanted, consequently generating major infections.

As a result of even a minimum microbial contamination and the consequent subliminal infection of the tissues, not detectable in the immediate intraoperative or early postoperative days, a hyperplastic inflammatory process is often established, which causes pain and edema at the site of the surgery or implant, with significant cicatrization and the formation of strong adherences that almost always require revision and removal of the implant.

Recent studies indicate that peri-prosthetic infections are the foremost reason for failure for knee prostheses and the third reason for hip prostheses in the United States. Also in Europe, with slight differences between the countries, peri-prosthetic infections are among the first three reasons that lead to revision surgery. In specialized centres, the current overall risk of peri-prosthetic infection is relatively low, from 0.5 to 3%, some conditions, however, can increase the risk. These comprise: previous interventions on the same joint, diabetes, smoking, alcohol or drugs, peripheral vasculopathies, kidney failure, corticosteroid treatment. The occurrence of two or more risk factors in the same patient, increases the risk exponentially. Furthermore, infection occurs on average in 2% of cases, even with the presence of optimal asepsis, of a correct surgical procedure and adequate antibiotic prophylaxis.

In Italy alone, according to recent statistics, over 150,000 primary prostheses per year are effected (65% of the hip, 31% of the knee) with an infection percentage ranging from 0.5-2% for the hip and from 2.5-5% (North) to 0.6-12% (Centre-South-Islands) for the knee. The percentage of infection for re-implants varies from 2.5% to 20%. The bacteria most frequently involved are *Staphylococcus epidermidis* (36% of infectious disease cases on prostheses), *Staphylococcus aureus* and *MRSA Methicillin-Resistant Staphylococcus aureus* (29%), plurimicrobial flora (16%). Also in subsequent percentages: anaerobic bacteria (5%), *Enterococcus faecalis* (4%), *Escherichia coli* (3%), *Pseudomonas species (spp.)* (2%).

Peri-prosthetic infections can be classified on the basis of the onset time of the symptoms after the implant, and can be subdivided as follows:

(a) early infections with an onset time ranging from 4 weeks to 3 months after the implant, which are believed to be acquired during surgery due to the dispersion of microbial material on the operative field, or, more rarely, can be linked to other infectious outbreaks already present and not eradicated prior to the intervention;

(b) late infections with an onset time ranging from 3 to 24 months after the implant, which are considered as being an exogenous acquisition and, as it is assumed that the biofilm is now well structured, the most shared therapeutic route is represented by a combined medical-surgical approach involving the removal of the prosthesis with positioning of the spacer cement and re-positioning of the prosthesis once it has been asserted that the infection has been eradicated;

(c) delayed infections which arise 24 months after the implant and are considered as being hematogenic pathogenesis from remote infection sites, whose treatment depends on the precocity of the diagnosis and therefore the formation or non-formation of a structured biofilm.

Various methods have been proposed for the antimicrobial surface treatment of implantable medical devices, aimed at minimizing the risk of microbial proliferation.

Various technologies are being studied or are already on the market, among which the following can be mentioned:

1. PSM (Passive Surface finishing/Modification): inert coatings that do not release bactericidal substances in the surrounding tissues, but that are produced so as to prevent or reduce bacterial adhesion thorough chemical substrates and/or structural modifications.

2. ASM (Active Surface finishing/Modification): biologically active coatings characterized by consisting of pharmacologically active pre-incorporated antibacterial agents.

3. LCC (Local Carriers or Coatings): local carriers or coatings of antibiotics, biodegradable or not, administered at the time of the surgical procedure, immediately before or at the same time as the implant.

The classification of the various technologies is useful for being able to better compare different solutions and improve the design of validation tests, in order to optimize the regulatory processes in this rapidly evolving field.

In the article of Visai et al, Int. J. Artif. Organs (2011) (9) :929-946, for example, prostheses made of titanium, or its alloys, are surface treated by means of electrochemical methods with the formation of a coating of titanium dioxide with an anatase structure, capable of being activated with suitable UV-Vis radiation in order to generate free radicals. This leads to the development of antiseptic and hydrophilic properties on the surface of the prosthesis. The hydrophilic properties are preserved for a certain period of time even after the implant, stimulating a specific cellular action *in situ,* that can facilitate bone integration.

The photosensitivity of the anatase and resulting antiseptic properties, however, cease once the prosthesis has been implanted, as the activation action of the UV-Vis radiation obviously ceases. According to some authors, moreover, the antiseptic and antibacterial action of the anatase coating exerts a toxic action with respect to the osteoblasts hindering the formation of new bone. Reference should be made, for example, to the articles of Arenas M.A. et al, Colloids Surf B: Biointerfaces (2013) 105:106-12 and Hu H. et al, Acta Biomater. (2012) 8:904-15.

Coatings with anatase, having characteristics far removed from those of biogenic materials, have recently given way to the construction of prostheses with biomimetic coatings, which mimic the bone tissue as much as possible, with respect to composition, morphological structure and mechanical properties, and to which antimicrobial properties can be imparted.

In the article of M. Stigter et al, J. Controlled Release, Vol. 99, Issue 1 (2004), 127-137, for example, the application of a coating of hydroxyapatite carbonate (CHA) is described, on titanium implants by means of a biomimetic precipitation method in which various antibiotics are incorporated, in particular cephalothin, carbenicillin, amoxicillin, cefamandol, tobramycin, gentamycin and vancomycin, which are released *in situ*.

Patent US 5.217.493, on the other hand, describes the use of specific mixtures of antibiotics (rifampin and minocycline or rifampin and novobiocin) for the surface treatment of implantable medical devices by simple immersion in a solution containing antibiotics, possibly with the aid of surfactants or other materials suitable for creating a bond between the antibiotic molecules and the surface of the device.

Patent US 6.596.338 describes a process for the coating of a metal prosthesis with a layer of calcium phosphate, which is effected by simple immersion in an aqueous solution from which the calcium phosphate precipitates. The coating can be effected by consecutive depositions of calcium phosphate, wherein the first layer has a thickness ranging from 0.01 $\mu$m to 10 $\mu$m, whereas the subsequent layers have a thickness ranging from 1 $\mu$m to 40 $\mu$m. The coating contains an active ingredient, in particular an antibiotic, which can be added to the calcium phosphate before implanting the prosthesis, or it can be absorbed by the coating layer at the time of the implant by immersion in an aqueous solution of the antibiotic.

With respect to the use of antibiotics for preventing the proliferation of microorganisms, it should be noted that scientific studies have shown that this use in the orthopaedic field generates a resistance on the part of the organism against the antibiotics themselves and the active ingredients used for limiting bacterial proliferation on the surface of the metal prosthesis used in implantology.

Again, in order to limit microbial proliferation on the surface of prosthetic implants, various techniques are known for producing silver-doped hydroxyapatite coatings. In this respect, reference should be made, for example, to the article of W. Chen et al, Biomaterials, 27 (2006), 5512-5517, in which the deposition of hydroxyapatite and silver ions on titanium disks is effected with co-pulverization (cosputtering) techniques that require the use of a magnetron.

The article of V. Stanic et al, Applied Surface Science, 256 (2010), 6083-6089, describes the preparation of nanopowders of hydroxyapatite doped with copper and zinc, which have antimicrobial properties that would suggest its use in various sectors, among which the treatment of implants in the orthopaedic field.

In this respect, it should be noted that the negative effects of silver are known, which, when put in prolonged contact with the skin, even in small doses, can cause serious damage to the organism due to its high toxicity. The toxic effects of copper which is an extremely toxic heavy metal with respect to living organisms, even in very small doses, are also known. MICHELE IAFISCO ET AL: "Adsorption and spectroscopic characterization of lactoferrin on hydroxyapatite nanocrystals",DALTON TRANSACTIONS, vol. 40, no. 4, 1 January 2011 (2011-01-01), page 820, discloses an antimicrobial material comprising nanocrystalline hydroxyapatite (HA) functionalized with lactoferrin which can be used in coating for implantable bone devices.

Finally, Italian patent IT 1.402.560 describes a process for coating a metal prosthetic implant in order to obtain a high osseointegration of the implant itself with the receiving bone structure, which comprises the electrodeposition on the surface of the implant of a nanometric layer of hydroxyapatite and a subsequent thermal treatment at a temperature ranging from 70°C to 800°C in order to increase the adhesion of the apatite nanocrystals to the surface of the implant.

The Applicant has considered the problem of providing implantable medical devices, in particular metal prostheses, with a coating capable of guaranteeing a high antimicrobial effect, in particular with respect to the main bacterial reference strains, which is exerted not only at the moment of insertion of the implant, but also when the implant has been completed, as, as illustrated above, the risk of the development of infection remains. This coating, moreover, must not represent an obstacle for the osseointegration of the prosthesis, but, on the contrary, must have biomimetic properties which are such as to favour osseointegration processes and remain perfectly attached to the prosthesis without causing extremely dangerous detachments for the stability and duration of the prosthesis itself. Furthermore, the coating must not contain products that can have harmful or even toxic effects for the patient.

This problem is solved according to the present invention by a coating of the implantable medical device as defined in the following description and enclosed claims.

According to a first aspect, the present invention therefore relates to an implantable medical device having a coating layer comprising a nanostructured hydroxyapatite doped with zinc ions and functionalized with lactoferrin.

Nanostructured hydroxyapatite is believed to confer the desired osseointegration properties, whereas the association of zinc ions and lactoferrin exerts a strong antimicrobial effect. Zinc ions, in fact, have marked antibacterial properties without any negative effect from a biological point of view, as zinc is a trace element present in living beings and essential for the cell metabolism. Lactoferrin, on the other hand, is a glycoprotein (molecular weight equal to about 80 kDa), present in various biological fluids and in the milk of mammals, which intervenes in the iron metabolism and is capable of influencing the development of microorganisms just thanks to its binding properties with respect to the iron ions, essential for microbial growth. The Applicant has found that lactoferrin exerts a potentiating effect of the antimicrobial activity of zinc ions and that this effect is particularly evident when zinc ions are included in a hydroxyapatite as doping agents, in partial substitution of calcium ions, which is functionalized with lactoferrin.

Without intending to be bound to any interpretative theory, it is believed that lactoferrin is capable of functionalizing hydroxyapatite by binding itself to the surface of the latter by various kinds of interactions, which can be either covalent bonds, or weaker bonds, such as electrostatic interactions, hydrogen bonds or van der Waals bonds.

According to a particularly preferred aspect, the implantable medical device is at least partially composed of a metal material and the nanostructured hydroxyapatite doped with zinc ions is obtained by electrodeposition on the surface of said metal material. This process, as better illustrated hereunder, allows a layer of nanostructured hydroxyapatite with a reduced thickness to be obtained, consisting of nanocrystals having dimensions and form which are such as to guarantee a high biomimetic effect, i.e. a morphology which imitates that typical of bone tissue and therefore favours osseointegration. The nanocrystals preferably have a lanceolate or roundish shape, which make them similar to the hydroxyapatite crystals present in the bone tissue.

The nanostructured hydroxyapatite doped with zinc ions is preferably composed of nanocrystals having an average size (x and y) lower than or equal to 400 nm, more preferably from 10 nm to 200 nm. The nanocrystals more preferably have an average length ranging from 10 nm to 200 nm and an average width ranging from 10 nm to 100 nm. The nanocrystals can be possibly at least partially aggregated to form "clusters" generally having micrometric dimensions, in particular ranging from 0.2 $\mu$m to 10 $\mu$m, preferably from 0.5 $\mu$m to 2 $\mu$m.

Within the scope of the present description and enclosed claims, generally the average particle dimensions are determined, preferably with respect to clusters, by means of dynamic light scattering (DLS) with laser source, see for example the standard ISO 13320-2009), or, preferably with respect to nanocrystals, by means of image analysis obtained with a transmission electron microscope (TEM).

As indicated above, the nanostructured hydroxyapatite is doped with zinc ions, i.e. the zinc ions partially substitute the calcium ions. The zinc ions are preferably present in a quantity which is such as to obtain a substitution degree of calcium ions ranging from 0.01% to 10% by weight, more preferably from 0.1% to 5% by weight, with respect to the total content of calcium ions.

Preferably, the nanostructured hydroxyapatite further comprises carbonate ions which partially substitute the phosphate ions. The carbonate ions are preferably present in a quantity which is such as to obtain a substitution degree of phosphate ions ranging from 0.1% to 10% by weight, more preferably from 0.5% to 5% by weight, with respect to the total content of phosphate ions.

In a further preferred embodiment, the nanostructured hydroxyapatite doped with zinc ions has the following formula:

$$Ca_{10-x} Zn_x (PO_4)_{6-y} (CO_3)_{y+z} (OH)_2$$

wherein:

x is a number ranging from 0.0055 to 0.6;
y is a number ranging from 0.065 to 0.9.

The nanostructured hydroxyapatite doped with zinc ions preferably has a crystallinity degree (CD) ranging from 15% to 50%, more preferably ranging from 20% to 40%.

The crystallinity degree (CD) can be determined using methods well known to skilled persons in the field, such as, for example, by X-ray diffraction data analysis. In particular, the crystallinity degree (CD) is measured according to the method described in Landi, E., Tampieri, A., Celotti, G., Sprio, S., "Densification behaviour and mechanisms of synthetic hydroxyapatites", J. Eur. Ceram. Soc., 20 (2000), 2377-2387:

$$CD = (1-X/Y) \bullet 100$$

wherein:

Y = maximum diffraction height at $2\theta = 33°$;
X = background diffraction height at $2\theta = 33°$
of the X-ray diffraction patterns of the nanoparticles.

As regards lactoferrin, this is a protein available on the market and can be obtained by separation and purification from products naturally containing it (for example milk), or by means of genetic engineering techniques (recombinant DNA).

The coating layer according to the present invention preferably comprises: from 85% by weight to 99.99% by weight, more preferably from 90% by weight to 99.9% by weight, of nanostructured hydroxyapatite doped with zinc ions; and from 0.01% by weight to 15% by weight, more preferably from 0.1% by weight to 10% by weight, of lactoferrin, with respect to the total weight of the coating layer.

The coating layer preferably has an average thickness ranging from 10 nm to 500 nm, more preferably from 50 nm to 200 nm.

As far as the implantable medical device is concerned, this is preferably a metal prosthetic device having a porous surface, which can be implanted in replacement interventions of large joints, for example hip or knee, or osteosynthesis. These devices generally consist of titanium or its alloys, in particular titanium/vanadium, titanium/aluminum or titanium/vanadium/aluminum alloys. Titanium alloys with 6% of Al and 4% of V (Ti6A14V ELI) are particularly preferred.

The metal prosthetic devices generally have a porous surface in order to favour bone attachment and a rapid vascularization. The porosity generally ranges from 20% to 80% by volume, whereas the average pore size preferably ranges from 100 $\mu$m to 500 $\mu$m, more preferably from 150 $\mu$m to 450 $\mu$m. These parameters can be determined according to known techniques, for example by means of SEM image analysis of a transversal section of the device (see for example EP 2 130 516 A1).

The surface porosity can be obtained in various ways, in particular:

(a) by random sintering of pure titanium microparticles (CPTi - commercially pure titanium), which, upon stratifying, generate a three-dimensional surface with a porosity of about 63% and an average pore diameter of about 300 $\mu$m, ideal for promoting bone regrowth, obtained for example according to the GRIPTION-STAT technique (Super Textured Asperity Topography) of DePuy Orthopedics Inc., Warsaw, USA;
(b) by sintering with the SLM (Selective Laser Melting) technique, with which a trabecular titanium surface is produced, having a high roughness with irregularly shaped pores (average porosity of 55% and an average pore diameter of about 500 $\mu$m), obtained for example according to the TRASER technique of Permedica S.p.A.

The surface porosity can also be obtained by laser sculpture of the metal surface to obtain a sponge structure.

In a second aspect, the present invention relates to a first process for coating an implantable medical device, which comprises:

providing an electrolytic bath comprising calcium ions, phosphate ions, zinc ions and lactoferrin;
immersing a cathode and an anode in said electrolytic bath, said cathode comprising the implantable medical device;
passing a direct electric current through said electrolytic bath so as to obtain an electrolytic deposition on the implantable medical device of a nanostructured hydroxyapatite doped with zinc ions and functionalized with lactoferrin.

In a third aspect, the present invention relates to a second process for coating an implantable medical device, which comprises:

providing an electrolytic bath comprising calcium ions, phosphate ions and zinc ions;
immersing a cathode and an anode in said electrolytic bath, said cathode comprising the implantable medical device;

passing a direct electric current through said electrolytic bath so as to obtain an electrolytic deposition on the implantable medical device of a nanostructured hydroxyapatite doped with zinc ions;

depositing lactoferrin on the medical implantable device coated with the nanostructured hydroxyapatite doped with zinc ions.

Consequently, according to the first process, the lactoferrin is combined with the hydroxyapatite during the production of the latter, and a more complete functionalization of the latter with the protein is therefore obtained, which is essentially distributed throughout the whole thickness of the coating.

According to the second process, on the other hand, the lactoferrin is deposited on the coating of hydroxyapatite only when the latter has already been formed, and consequently the lactoferrin is only present on the surface. A double-layered coating is therefore obtained: a first inner layer of hydroxyapatite without lactoferrin and a second outer layer in which the hydroxyapatite is functionalized with lactoferrin.

Some preferred process conditions are indicated hereunder, which can be applied to both of the processes described above, unless otherwise specified.

The implantable medical device is preferably at least partially composed of a metal material.

The electrolytic bath is preferably kept at a temperature ranging from 5°C to 50°C, preferably from 20°C to 30°C.

The calcium ions are added to the electrolytic bath by means of a calcium salt soluble in water under the reaction conditions, for example $Ca(NO_3)_2*4H_2O$, $Ca(NO_3)2*2H_2O$, $Ca(CH_3COO)_2$, or mixtures thereof. The initial concentration of the calcium ions in the electrolytic bath generally ranges from 0.01 to 0.1 moles/litre, preferably from 0.02 to 0.06 moles/litre.

The phosphate ions are added to the electrolytic bath by means of a phosphate salt soluble in water under the reaction conditions, for example $NH_4H_2PO_4$, $K_2HPO_4$, $KH_2PO_4$, $H_3PO_4$, or mixtures thereof. The initial concentration of the phosphate ions in the electrolytic bath generally ranges from 0.01 to 0.10 moles/litre, preferably from 0.02 to 0.04 moles/litre.

The zinc ions are added to the electrolytic bath by means of a zinc salt soluble in water under the reaction conditions, for example $ZnCl_2$, $Zn(CH_3COO)_2$, $Zn(NO_3)_2$, or mixtures thereof. The initial concentration of the zinc ions in the electrolytic bath generally ranges from 0.001 to 0.30 moles/litre, preferably from 0.01 to 0.05 moles/litre.

According to a preferred embodiment, at the beginning of the deposition, the electrolytic bath has a pH ranging from 3.0 to 4.5, preferably from 3.5 to 4.0, said pH increasing near the cathode to a value higher than 8, generally ranging from 10 to 12, during the deposition process without the addition of alkaline compounds. At the end of the electrodeposition process, the electrolytic bath has generally a pH ranging from 4.0 to 6.0, preferably from 4.5 to 5.5.

According to the first process, the lactoferrin is added to the electrolytic bath, for example in the form of an aqueous solution. The initial concentration of the lactoferrin in the electrolytic bath generally ranges from 0.001 to 0.3 mg/mL, preferably from 0.01 to 0.1 mg/mL.

The use of lactoferrin allows a coating to be obtained, consisting of nanocrystals of hydroxyapatite doped with zinc ions and functionalized by protein, which is capable of providing the bone surface with an interface not purely inorganic, but biologically hybrid that can exert a high antibacterial effect. According to the process defined above, the lactoferrin is inserted in the electrodeposition process of the hydroxyapatite coating inside the electrolytic bath: in this way, the growth of hydroxyapatite nanocrystals doped with zinc ions is obtained on the metal surface, in the presence of lactoferrin, which is embedded in the same coating.

According to the second process defined above, on the other hand, the lactoferrin can be deposited on the hydroxyapatite coating already formed, for example by simple immersion of the same in an aqueous solution of lactoferrin. In this way, the functionalization of the hydroxyapatite by means of lactoferrin is substantially only on the surface. A hybrid coating is thus obtained, consisting of a hydroxyapatite phase and a surface biopolymeric phase.

According to a preferred embodiment, the electrodeposition is carried out with a substantially constant direct current. The direct current is preferably kept at relatively low values to avoid detachments of the material deposited caused by an excessive development of gaseous hydrogen at the cathode. The direct current generally has an intensity ranging from 10 mA to 100 mA, more preferably from 20 mA to 60 mA.

The potential of the applied current can range from 5 V to 150 V, but preferably from 20 V to 80 V.

Preferably, the coating of the implantable medical device is effected through a plurality of electrolytic deposition steps of the nanostructured hydroxyapatite carried out in succession (pulsed electrochemical process). These steps are carried out for a relatively short period of time and they are separated by short breaks in which the passage of current is interrupted. In this way, the homogeneity and adhesion of the overall coating layer are improved, as an excessively lengthy electrolytic deposition step can lead to a significant development of hydrogen on the surface to be coated, which creates defects and unevenness in the coating material.

Each electrolytic deposition step preferably has a duration ranging from 3 seconds to 60 seconds, preferably from 10 seconds to 20 seconds. The subsequent electrolytic deposition steps are separated by a pause in which the passage of current is interrupted, whose duration can range from 1 second to 1 minute, preferably from 1 second to 10 seconds.

The total duration of the electrolytic deposition process mainly depends on the thickness of the coating to be produced and on the process conditions, and generally ranges from 40 seconds to 10 minutes, preferably from 1 minute to 5 minutes.

The number of deposition steps in succession generally ranges from 4 to 50, preferably from 6 to 30.

At the end of the electrochemical process, the medical device thus coated is extracted from the electrolytic bath and dried by a gas flow (generally air) at a temperature ranging from 20°C to 100°C, more preferably from 30°C to 50°C, for a time ranging from 100 minutes to 500 minutes.

The implantable medical device coated according to the present invention has high antibacterial characteristics without the use of synthesis compounds with an antibacterial effect (for example antibiotics such as vancomycin), and also without the use of substances having a toxic effect with respect to living organisms (such as the above-mentioned copper and silver), nor metal nanoparticles (also having a toxic effect), but with the use of ions of zinc, which is an important trace element, and lactoferrin, a protein of natural origin. These components are included in a nanostructure hydroxyapatite coating, having characteristics which are completely similar to those of the bone with which it will come into contact once the prosthesis has been implanted *in situ*.

The following working examples are provided for purely illustrative purposes of the present invention and should not be considered as limiting the protection scope defined by the enclosed claims.

EXAMPLE 1

55 mL of an aqueous solution of $Ca^{2+}$ ions at a concentration of 55 mM, 47 mL of an aqueous solution of $PO_4^{3-}$ ions at a concentration of 25 mM, 7 mL of a solution of $Zn^{2+}$ ions at a concentration of 20 mM, 7 mL of a solution of lactoferrin at a concentration of 0.02 mg/mL, were introduced into an electrolytic cell. After completely mixing the solutions, a platinum anode and a control electrode were introduced into the electrolytic cell. As cathode, a portion of hip prosthesis in titanium alloy, having a size of about 5 cm x 5 cm and a thickness of 6 mm, corresponding to a quarter of the hemisphere of an acetabular cup having a diameter of 64 mm of the prosthetic cotyledon Gription™ Pinnacle in titanium alloy (Ti6A14V) of DePuy, Warsaw, USA, was used as sample, positioned in front of the platinum anode. This prosthetic component was surface treated by sintering microparticles of CPTi (commercially pure titanium) according to the Gription™ technique (porosity 63%, average pore diameter about 300 µm).

A current intensity equal to 22 mA and a potential of 33 V were then applied in the electrochemical cell. The electrodeposition process was carried out at a temperature of 25°C, divided into four consecutive steps, each having a duration of 12 seconds. The passage of current was interrupted for 5 seconds between one step and the next one.

The two electrodes had comparable dimensions in order to avoid unevenness in the formation of the coating. The distance between the two electrodes varied from 1 cm to 3 cm, as the cathode had not a flat shape.

At the end of the process, the cathode composed of the portion of coated prosthesis was extracted from the electrochemical bath and dried under a flow of hot air at a temperature of 50°C, for a time equal to 120 minutes.

The coating thus obtained contained hydroxyapatite partially substituted with zinc ions in a percentage, with respect to the calcium ions, equal to 1.5%, and functionalized with 0.1% by weight of lactoferrin, with respect to the total weight of the coating.

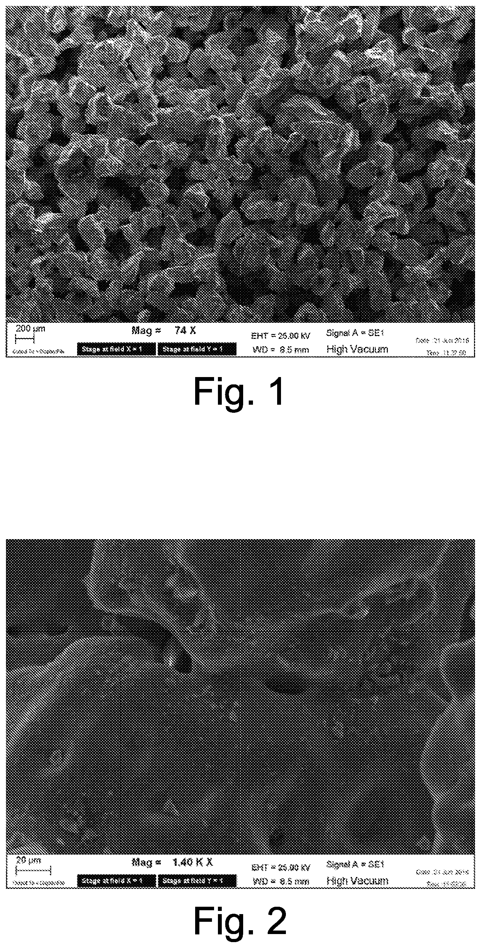

The enclosed figures 1-3 relate to:

Figure 1 - image with a scanning electron microscope (SEM) of the surface of the prosthesis before coating by electrodeposition;
Figure 2 - image with a scanning electron microscope (SEM) of the surface of the prosthesis after coating by electrodeposition: the presence of micrometric agglomerates is clearly visible;
Figure 3 - analysis spectrum obtained with an EDX probe of the surface of the prosthesis after coating by electrodeposition: the presence of calcium (Ca) and phosphorous (P) in the ratios typical of hydroxyapatite, can be observed, the signals typical of titanium (Ti) and aluminium (Al) which form the alloy of which the prosthesis is composed, are also present.

EXAMPLE 2

40 mL of an aqueous solution of $Ca^{2+}$ ions at a concentration of 42 mM, 40 mL of an aqueous solution of $PO_4^{3-}$ ions at a concentration of 25 mM, 4 mL of a solution of $Zn^{2+}$ ions at a concentration of 42 mM, were introduced into the same electrolytic cell as Example 1. After completely mixing the solutions, a platinum anode and a control electrode were introduced into the electrolytic cell. An identical portion of hip prosthesis in titanium alloy as described in Example 1, was used as cathode, positioned in front of the platinum anode.

A current intensity equal to 20 mA and a potential of 30 V were then applied in the electrochemical cell. The electrodeposition process was carried out at a temperature of 25°C, divided into six consecutive steps, each having a duration of 10 seconds. The passage of current was interrupted for 10 seconds between one step and the next one.

The two electrodes had comparable dimensions in order to avoid unevenness in the formation of the coating. The distance between the two electrodes varied from 1 cm to 3 cm, as the cathode had not a flat shape.

At the end of the process, the cathode composed of the sample of coated prosthesis was extracted from the electrochemical bath and immersed in a solution of lactoferrin with a concentration equal to 0.059 mg/mL for a time equal to 50 seconds. After extraction from the solution, the prosthesis was dried under a flow of hot air at a temperature of 40°C, for a time equal to 300 minutes.

The coating thus obtained contained hydroxyapatite partially substituted with zinc ions in a percentage, with respect to the calcium ions, equal to 2.0%, and functionalized with 0.2% by weight of lactoferrin, with respect to the total weight of the coating.

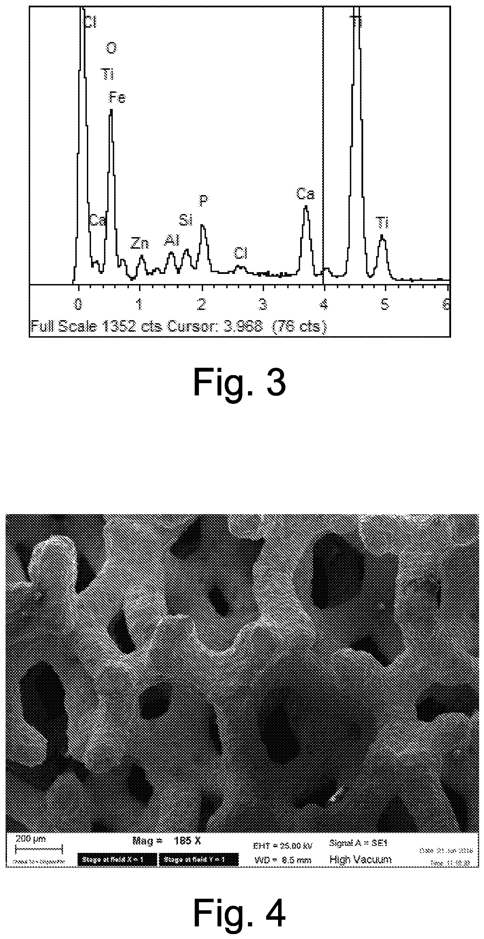

The attached figures 4-5 relate to:

Figure 4 - image with a scanning electron microscope (SEM) of the surface of the prosthesis after coating by electrodeposition;

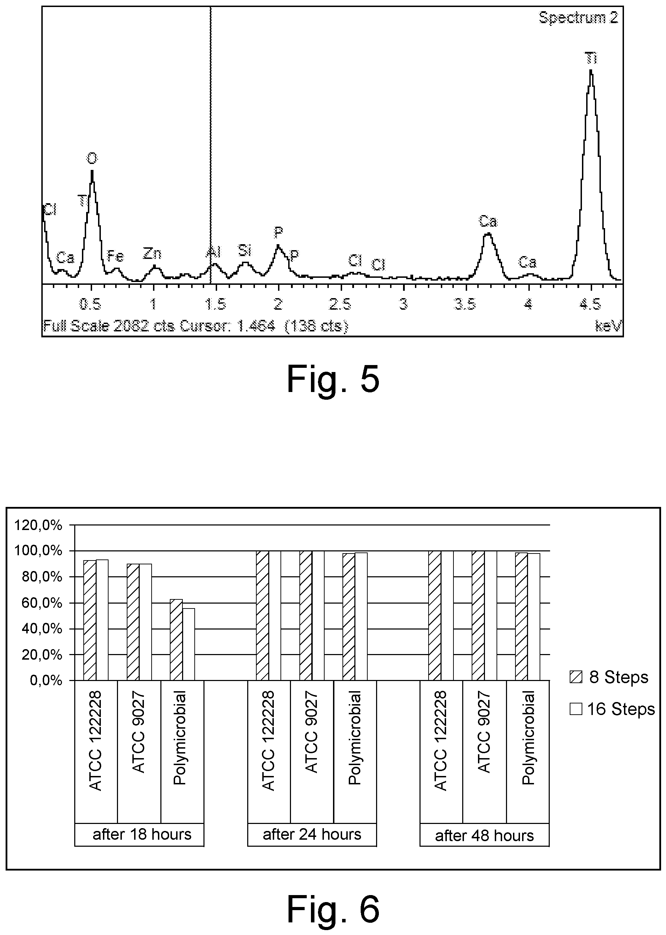

Figure 5 - analysis spectrum obtained with an EDX probe of the surface of the prosthesis after coating by electrodeposition: the presence of calcium (Ca) and phosphorous (P) in the ratios typical of hydroxyapatite, can be observed, the signals typical of titanium (Ti) and aluminium (Al) which form the alloy of which the prosthesis was composed, are also present.

EXAMPLES 3-4 (comparative)

Example 1 was repeated using the same conditions indicated above, except that: in Example 3, the final coating layer contained hydroxyapatite doped with zinc ions but was devoid of lactoferrin, which had not been added either to the electrolytic bath or after electrolytic deposition; in Example 4, the final coating layer contained hydroxyapatite functionalized with lactoferrin but was devoid of zinc ions, as these had not been added to the electrolytic bath.

EXAMPLE 5: microbiological tests

The samples of coated metal prosthesis according to Examples 1, 3 and 4 were evaluated for antimicrobial activity with respect to contamination *in vitro* with various bacteria, monitoring this activity at increasing time intervals from the moment of contamination. The bacterial growth was verified under aerobic conditions at 37°C.

The determination of the antibacterial activity was effected as described in the Standard Method ASTM E2180:2007. The method involves comparing the inhibiting activity of the sample under examination with an equivalent sample not surface treated and therefore without antibacterial agent (control).

The samples under examination were sterilized in an autoclave (NUARE autoclave at 121°C, 1 atm for 15 min), inoculated with the microbial strain (400 $\mu$L) on the porous surface destined for coming into contact with the bone tissue, covered with film, inserted in a Petri plate and introduced into an incubator at a temperature of 37 $\pm$ 1 °C. The determination of the residual vital bacterial charge was effected after increasing incubation times: 24 hours, 48 hours, 7 days, 15 days, 21 days. The values indicated hereunder are the average of three identical samples for each bacterial strain. At the end of the incubation period, in order to avoid interferences in the microbiological result, the effect of the antibacterial activity was annulled by immerging the sample in a neutralizing solution, consisting of: 3 g of lecithin, 30 g of polysorbate 80, 5 g of sodium thiosulfate, 1 g of L-histidine, distilled water to 100 ml.

The contamination of the samples was effected using the following bacterial strains:

(A) *Staphylococcus epidermidis* (SCN -Gram+ bacterium) (ATCC 12228);
(B) Polymicrobial inoculum consisting of a combination of anaerobic bacteria (Propionibacterium species) and facultative aerobic/anaerobic bacteria (*Staphylococcus MRSA, Pseudomonas aeruginosa* and *Enterococcus faecalis*).

The bacterial contamination is expressed as CFU/ml, wherein CFU = Colony Forming Units.

The percentage reduction of bacterial vitality (R) was determined for each sample and bacterial strain, according to the following formula:

$$R = [(A - B) / A] * 100$$

wherein:

A is the logarithm of the bacterial count on the control sample;

B is the logarithm of the bacterial count on the sample under examination at the end of the preestablished time.

Each value of A and B is the average value on the basis of three samples.

The results are indicated in Table 1 hereunder:

TABLE 1

| Sample | Bacterial inoculum | after 24 h | after 48 h | after 7 days | after 15 days | after 21 days |
|---|---|---|---|---|---|---|
| Ex. 1 (HA+Zn+LTF) | *Staphylococcus epidermidis* | 42% | 47% | 90% | 98% | 98% |
| Ex. 1 (HA+Zn+LTF) | Polymicrobial | 50% | 52% | 83% | 97% | 98% |
| Ex. 3 (HA+Zn) | *Staphylococcus epidermidis* | 15% | 17% | 73% | 85% | 86% |
| Ex. 3 (HA+Zn) | Polymicrobial | 28% | 28% | 66% | 78% | 83% |
| Ex. 4 (HA+LTF) | *Staphylococcus epidermidis* | 24% | 42% | 85% | 88% | 89% |
| Ex. 4 (HA+LTF) | Polymicrobial | 38% | 43% | 79% | 83% | 83% |
| HA : hydroxyapatite LTF : lactoferrin | | | | | | |

From the data indicated above, it is evident that the surface treatment with HA+Zn+LTF allows a much greater reduction in microbial growth with respect to the surface treatments with HA+Zn and HA+LTF, with both the St. *Epidermidis* inoculum and also with polymicrobial inoculum. The improved antimicrobial effect is already evident after 24 hours of incubation, it increases with time and becomes almost complete (97-98%) with HA+Zn+LTF after 15 and 21 days of incubation.

EXAMPLES 6-7

Example 1 was repeated with the same materials and the same operating conditions described above, except that the electrodeposition process was carried out in eight (Example 6) or sixteen (Example 7) consecutive steps, each having a duration of 13 seconds (interval of 5 seconds between one step and the next one).

The samples of titanium prosthesis thus coated were evaluated for the antimicrobial activity with respect to contamination *in vitro* with the following bacterial strains:

(A) *Staphylococcus epidermidis* (SCN -Gram+ bacterium) (ATCC 12228);
(B) Pseudomonas aeruginosa (Gram- bacterium) (ATC 9027) ;
(C) Polymicrobial inoculum consisting of a combination of anaerobic bacteria (Propionibacterium species) and facultative aerobic/anaerobic bacteria (*Staphylococcus MRSA, Pseudomonas aeruginosa* and *Enterococcus faecalis*).

The determination of the antibacterial activity was carried out according to what is described in the Standard Method ASTM E2180:2007, under the same conditions indicated in Example 5. The determination of the residual vital bacterial charge was effected after increasing incubation times: 18 hours, 24 hours and 48 hours.

The results are indicated in Table 2 hereunder:

TABLE 2

| Sample | Bacterial inoculum | after 18 hours | after 24 hours | after 48 hours |
|---|---|---|---|---|
| Ex..6 (HA+Zn+LTF) | *Staphylococcus epidermidis* | 92.3% | 99.6% | 99.97% |
| Ex. 7 (HA+Zn+LTF) | *Staphylococcus epidermidis* | 93.1% | 99.4% | 99.90% |
| Ex. 6 (HA+Zn+LTF) | *Pseudomonas aeruginosa* | 90.0% | 99.7% | 99.99% |
| Ex. 7 (HA+Zn+LTF) | *Pseudomonas aeruginosa* | 90.0% | 99.4% | 99.98% |
| Ex. 6 (HA+Zn+LTF) | Polymicrobial | 63.0% | 98.6% | 99.00% |
| Ex. 7 (HA+Zn+LTF) | Polymicrobial | 55.6% | 98.25% | 99.00% |

The data of Table 2 are graphically indicated in the enclosed Figure 6. They show that, even for short contact times (24 hours), the coating according to the present invention, obtained with an electrodeposition process with 8 or 16 consecutive steps, allows a percentage reduction in the bacterial vitality higher than 99% to be obtained with respect to both Gram positive bacteria (*Staphylococcus epidermidis*) and Gram negative bacteria (*Pseudomonas aeruginosa*). As far as the polymicrobial charge is concerned, after 24 hours of contact, a reduction of about 98% was observed, mainly due to an increase in the value of the control sample.

The reduction in the bacterial vitality was established after 48 hours as being at values close to 100%, i.e. an almost complete disappearance of the bacteria, both Gram positive (*Staphylococcus epidermidis*) and Gram negative (*Pseudomonas aeruginosa*). Analogous values, around 99%, were obtained with the polymicrobial charge.

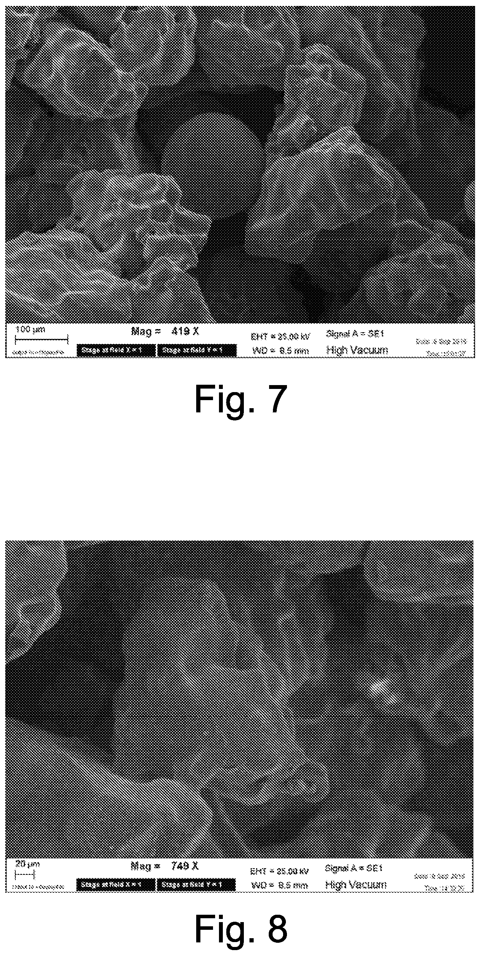

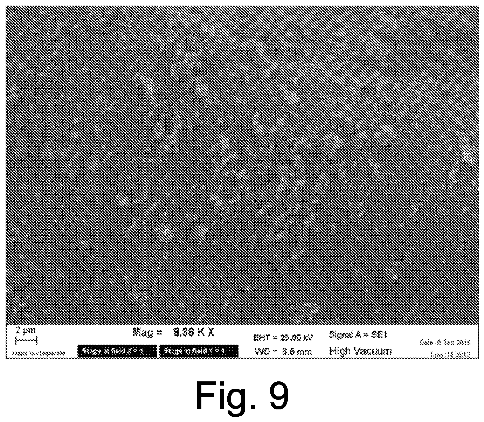

The possible presence of non-homogeneity or even detachments of the coating layer was verified by effecting SEM microphotographs of the samples according to Example 7, which are indicated in the attached Figures 7-9. As can be observed from these microphotographs, the coating layer is homogeneous and adheres perfectly to the surface of the prosthesis.

**Claims**

1.  Implantable medical device having a coating layer comprising a nanostructured hydroxyapatite doped with zinc ions and functionalized with lactoferrin.

2.  Implantable medical device according to claim 1, at least partially consisting of a metal material and the nanostructured hydroxyapatite doped with zinc ions is obtained by electrodeposition on the surface of said metal material.

3.  Implantable medical device according to any one of the preceding claims, wherein the nanostructured hydroxyapatite doped with zinc ions consists of nanocrystals having an average size (x and y) lower than or equal to 400 nm, preferably from 10 nm to 200 nm.

4.  Implantable medical device according to any one of the preceding claims, wherein the zinc ions are present in an amount such that to obtain a substitution degree of the calcium ions from 0.01% to 10% by weight, preferably from 0.1% to 5% by weight, with respect to the total content of calcium ions.

5.  Implantable medical device according to any one of the preceding claims, wherein the nanostructured hydroxyapatite further comprises carbonate ions which partially substitute the phosphate ions.

6.  Implantable medical device according to any one of the preceding claims, wherein the nanostructured hydroxyapatite doped with zinc ions has a crystallinity degree (CD) comprised between 15% and 50%, preferably comprised between 20% and 40%.

7.  Implantable medical device according to any one of the preceding claims, wherein the coating layer comprises: from 85% by weight to 99.99% by weight, preferably from 90% by weight to 99.9% by weight, of nanostructured hydroxyapatite doped with zinc ions; and from 0.01% by weight to 15% by weight, preferably from 0.1% by weight to 10% by weight, of lactoferrin, with respect to the total weight of the coating layer.

8.  Implantable medical device according to any one of the preceding claims, wherein the coating layer has an average thickness from 10 nm to 500 nm, preferably from 50 nm to 200 nm.

9.  Implantable medical device according to any one of the preceding claims, wherein said device is a metal prosthetic device having a porous surface, preferably having a porosity from 20% to 80% by volume.

10.  Implantable medical device according to claim 9, wherein the average size of the pores is comprised between 100 $\mu$m and 500 $\mu$m, preferably comprised between 150 $\mu$m and 450 $\mu$m.

11.  Process for coating an implantable medical device according to any one of the claims from 1 to 10, comprising:

       providing an electrolytic bath comprising calcium ions, phosphate ions, zinc ions and lactoferrin;
       immersing a cathode and an anode in said electrolytic bath, said cathode comprising the implantable medical device;
       allowing the direct electric current to pass through said electrolytic bath so as to obtain an electrolytic deposition on the implantable medical device of a nanostructured hydroxyapatite doped with zinc ions and functionalized with lactoferrin.

12. Process for coating an implantable medical device according to any one of the claims from 1 to 10, comprising:

providing an electrolytic bath comprising calcium ions, phosphate ions and zinc ions;
immersing a cathode and an anode in said electrolytic bath, said cathode comprising the implantable medical device;
allowing the direct electric current to pass through said electrolytic bath so as to obtain an electrolytic deposition on the implantable medical device of a nanostructured hydroxyapatite doped with zinc ions;
depositing lactoferrin on the medical implantable device coated with the nanostructured hydroxyapatite doped with zinc ions.

13. Process according to claim 11 or 12, wherein at the beginning of the deposition the electrolytic bath has a pH comprised between 3.0 and 4.5, preferably between 3.5 and 4.0, said pH increasing near the cathode to a value higher than 8, generally comprised between 10 and 12, during the deposition process without adding alkaline compounds.

14. Process according to any one of claims from 11 to 13, wherein the electrodeposition is carried out with a substantially constant direct current, with an intensity comprised between 10 mA and 100 mA, preferably between 20 mA and 60 mA.

15. Process according to any one of claims from 11 to 14, wherein a plurality of electrolytic deposition steps of the nanostructured hydroxyapatite are carried out in succession.


**Patentansprüche**

1. Implantierbare medizinische Vorrichtung mit einer Beschichtung, umfassend nanostrukturiertes, mit Zinkionen dotiertes und mit Lactoferrin funktionalisiertes Hydroxyapatit.

2. Implantierbare medizinische Vorrichtung nach Anspruch 1, mindestens teilweise bestehend aus einem metallischen Material und wobei das nanostrukturierte, mit Zinkionen dotierte Hydroxyapatit durch Elektroabscheidung auf der Oberfläche des metallischen Materials erhalten wird.

3. Implantierbare medizinische Vorrichtung nach einem der vorstehenden Ansprüche, wobei das nanostrukturierte, mit Zinkionen dotierte Hydroxyapatit aus Nanokristallen mit einer durchschnittlichen Größe (x und Y) von weniger oder gleich 400 nm, vorzugsweise von 10 nm bis 200 nm besteht.

4. Implantierbare medizinische Vorrichtung nach einem der vorstehenden Ansprüche, wobei die Zinkionen in einer solchen Menge vorhanden sind, dass ein Substitutionsgrad der Calciumionen von 0,01 Gew.-% bis 10 Gew.-%, vorzugsweise von 0,1 Gew.-% bis 5 Gew.-% in Bezug auf den Gesamtgehalt an Calciumionen erhalten wird.

5. Implantierbare medizinische Vorrichtung nach einem der vorstehenden Ansprüche, wobei das nanostrukturierte Hydroxyapatit ferner Carbonationen umfasst, die die Phosphationen teilweise substituieren.

6. Implantierbare medizinische Vorrichtung nach einem der vorstehenden Ansprüche, wobei das nanostrukturierte, mit Zinkionen dotierte Hydroxyapatit einen Kristallinitätsgrad (CD) zwischen 15 % und 50 %, vorzugsweise zwischen 20 % und 40 % aufweist.

7. Implantierbare medizinische Vorrichtung nach einem der vorstehenden Ansprüche, wobei die Beschichtung umfasst: von 85 Gew.-% bis 99,99 Gew.-%, vorzugsweise von 90 Gew.-% bis 99,9 Gew.-% nanostrukturiertes, mit Zinkionen dotiertes Hydroxyapatit; und von 0,01 Gew.-% bis 15 Gew.-%, vorzugsweise von 0,1 Gew.-% bis 10 Gew.-% Lactoferrin in Bezug auf das Gesamtgewicht der Beschichtung.

8. Implantierbare medizinische Vorrichtung nach einem der vorstehenden Ansprüche, wobei die Beschichtung eine durchschnittliche Dicke von 10 nm bis 500 nm, vorzugsweise von 50 nm bis 200 nm aufweist.

**9.** Implantierbare medizinische Vorrichtung nach einem der vorstehenden Ansprüche, wobei die Vorrichtung eine metallische Prothesenvorrichtung mit einer porösen Oberfläche ist, die vorzugsweise eine Porosität von 20 Vol.-% bis 80 Vol.-% aufweist.

**10.** Implantierbare medizinische Vorrichtung nach Anspruch 9, wobei die durchschnittliche Größe der Poren zwischen 100 $\mu$m und 500 $\mu$m, vorzugsweise zwischen 150 $\mu$m und 450 $\mu$m beträgt.

**11.** Verfahren zum Beschichten einer implantierbaren medizinischen Vorrichtung nach einem der Ansprüche 1 bis 10, umfassend:

Bereitstellen eines Elektrolysebades, umfassend Calciumionen, Phosphationen, Zinkionen und Lactoferrin;
Eintauchen einer Kathode und einer Anode in das Elektrolysebad, wobei die Kathode die implantierbare medizinische Vorrichtung umfasst;
Ermöglichen von direktem elektrischem Stromdurchgang durch das Elektrolysebad, um auf der implantierbaren medizinischen Vorrichtung eine elektrolytische Abscheidung eines mit Zinkionen dotierten und Lactoferrin funktionalisierten, nanostrukturierten Hydroxyapatits zu erhalten.

**12.** Verfahren zum Beschichten einer implantierbaren medizinischen Vorrichtung nach einem der Ansprüche 1 bis 10, umfassend:

Bereitstellen eines Elektrolysebades, umfassend Calciumionen, Phosphationen und Zinkionen;
Eintauchen einer Kathode und einer Anode in das Elektrolysebad, wobei die Kathode die implantierbare medizinische Vorrichtung umfasst;
Ermöglichen von direktem elektrischem Stromdurchgang durch das Elektrolysebad, um auf der implantierbaren medizinischen Vorrichtung eine elektrolytische Abscheidung eines nanostrukturierten, mit Zinkionen dotierten Hydroxyapatits zu erhalten;
Abscheiden von Lactoferrin auf der implantierbaren medizinischen Vorrichtung, die mit nanostrukturiertem, mit Zinkionen dotiertem Hydroxyapatit beschichtet ist.

**13.** Verfahren nach Anspruch 11 oder 12, wobei zu Beginn der Abscheidung das Elektrolysebad einen pH-Wert zwischen 3,0 und 4,5, vorzugsweise zwischen 3,5 und 4,0 aufweist, wobei der pH-Wert während des Abscheidungsverfahrens ohne Zugabe von alkalischen Verbindungen in der Nähe der Kathode auf einen Wert über 8, im Allgemeinen zwischen 10 und 12, ansteigt.

**14.** Verfahren nach einem der Ansprüche 11 bis 13, wobei die Elektroabscheidung bei einem im Wesentlichen konstanten direkten Strom mit einer Stärke zwischen 10 mA und 100 mA, vorzugsweise zwischen 20 mA und 60 mA ausgeführt wird.

**15.** Verfahren nach einem der Ansprüche 11 bis 14, wobei eine Mehrzahl von elektrolytischen Abscheidungsschritten des nanostrukturierten Hydroxyapatits nacheinander ausgeführt werden.

**Revendications**

**1.** Dispositif médical implantable ayant une couche de revêtement comprenant une hydroxyapatite nanostructurée dopée avec des ions zinc et fonctionnalisée avec de la lactoferrine.

**2.** Dispositif médical implantable selon la revendication 1, constitué au moins partiellement d'un matériau métallique et l'hydroxyapatite nanostructurée dopée par des ions zinc est obtenue par électrodéposition sur la surface dudit matériau métallique.

**3.** Dispositif médical implantable selon l'une quelconque des revendications précédentes, dans lequel l'hydroxyapatite nanostructurée dopée avec des ions zinc est constituée de nanocristaux ayant une taille moyenne (x et y) inférieure ou égale à 400 nm, préférablement de 10 nm à 200 nm.

**4.** Dispositif médical implantable selon l'une quelconque des revendications précédentes, dans lequel les ions zinc sont présents en une quantité telle à obtenir un degré de substitution des ions calcium de 0,01 % à 10 % en poids, préférablement de 0,1 % à 5 % en poids, par rapport à la teneur totale en ions calcium.

**5.** Dispositif médical implantable selon l'une quelconque des revendications précédentes, dans lequel l'hydroxyapatite nanostructurée comprend en outre des ions carbonate qui remplacent partiellement les ions phosphate.

**6.** Dispositif médical implantable selon l'une quelconque des revendications précédentes, dans lequel l'hydroxyapatite nanostructurée dopée avec des ions zinc a un degré de cristallinité (CD) compris entre 15 % et 50 %, préférablement compris entre 20 % et 40 %.

**7.** Dispositif médical implantable selon l'une quelconque des revendications précédentes, dans lequel la couche de revêtement comprend : de 85 % en poids à 99,99 % en poids, préférablement de 90 % en poids à 99,9 % en poids, d'hydroxyapatite nanostructurée dopée avec des ions de zinc ; et de 0,01 % en poids à 15 % en poids, préférablement de 0,1 % en poids à 10 % en poids, de lactoferrine, par rapport au poids total de la couche de revêtement.

**8.** Dispositif médical implantable selon l'une quelconque des revendications précédentes, dans lequel la couche de revêtement a une épaisseur moyenne de 10 nm à 500 nm, préférablement de 50 nm à 200 nm.

**9.** Dispositif médical implantable selon l'une quelconque des revendications précédentes, dans lequel ledit dispositif est un dispositif prothétique métallique ayant une surface poreuse, ayant préférablement une porosité de 20 % à 80 % en volume.

**10.** Dispositif médical implantable selon la revendication 9, dans lequel la taille moyenne des pores est comprise entre 100 $\mu$m et 500 $\mu$m, préférablement comprise entre 150 $\mu$m et 450 $\mu$m.

**11.** Procédé pour revêtir un dispositif médical implantable selon l'une quelconque des revendications 1 à 10, comprenant :

le fait de fournir un bain électrolytique comprenant des ions calcium, des ions phosphate, des ions zinc et de la lactoferrine ;
le faire d'immerger une cathode et une anode dans ledit bain électrolytique, ladite cathode comprenant le dispositif médical implantable ;
le fait de permettre au courant électrique direct de passer à travers ledit bain électrolytique de façon à obtenir un dépôt électrolytique sur le dispositif médical implantable d'une hydroxyapatite nanostructurée dopée avec des ions zinc et fonctionnalisée avec de la lactoferrine.

**12.** Procédé pour revêtir un dispositif médical implantable selon l'une quelconque des revendications 1 à 10, comprenant :

le fait de fournir un bain électrolytique comprenant des ions calcium, des ions phosphate et des ions zinc ;
le fait d'immerger une cathode et une anode dans ledit bain électrolytique, ladite cathode comprenant le dispositif médical implantable ;
le fait de permettre au courant électrique direct de passer à travers ledit bain électrolytique de façon à obtenir un dépôt électrolytique sur le dispositif médical implantable d'une hydroxyapatite nanostructurée dopée avec des ions zinc ;
le fait de déposer de la lactoferrine sur le dispositif médical implantable revêtu de l'hydroxyapatite nanostructurée dopée avec des ions de zinc.

**13.** Procédé selon la revendication 11 ou la revendication 12, dans lequel au début du dépôt le bain électrolytique a un pH compris entre 3,0 et 4,5, préférablement entre 3,5 et 4,0, ledit pH augmentant près de la cathode jusqu'à une valeur supérieure à 8, généralement comprise entre 10 et 12, pendant le procédé de dépôt sans ajout de composés alcalins.

**14.** Procédé selon l'une quelconque des revendications 11 à 13, dans lequel l'électrodéposition est réalisée avec un courant continu substantiellement constant, avec une intensité comprise entre 10 mA et 100 mA, préférablement entre 20 mA et 60 mA.

**15.** Procédé selon l'une quelconque des revendications 11 à 14, dans lequel une pluralité d'étapes de dépôt électrolytique de l'hydroxyapatite nanostructurée sont réalisées successivement.

200 μm    Mag = 74 X    EHT = 25.00 kV    Signal A = SE1    Date :21 Jun 2016
Stage at field X = 1    Stage at field Y = 1    WD = 8.5 mm    High Vacuum    Time :11:32:50

Fig. 1

20 μm    Mag = 1.40 K X    EHT = 25.00 kV    Signal A = SE1    Date :21 Jun 2016
Stage at field X = 1    Stage at field Y = 1    WD = 8.5 mm    High Vacuum    Time :11:33:56

Fig. 2

Fig. 3

Fig. 4

Fig. 5

Fig. 6

Fig. 7

Fig. 8

Fig. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5217493 A
- US 6596338 B
- IT 1402560
- EP 2130516 A1

**Non-patent literature cited in the description**

- **VISAI et al.** *Int. J. Artif. Organs,* 2011, vol. 9, 929-946
- **ARENAS M.A. et al.** *Colloids Surf B: Biointerfaces,* 2013, vol. 105, 106-12
- **HU H. et al.** *Acta Biomater.,* 2012, vol. 8, 904-15
- **M. STIGTER et al.** *J. Controlled Release,* 2004, vol. 99 (1), 127-137
- **W. CHEN et al.** *Biomaterials,* 2006, vol. 27, 5512-5517
- **V. STANIĆ et al.** *Applied Surface Science,* 2010, vol. 256, 6083-6089
- **MICHELE IAFISCO et al.** Adsorption and spectroscopic characterization of lactoferrin on hydroxyapatite nanocrystals. *DALTON TRANSACTIONS,* 01 January 2011, vol. 40 (4), 820
- **LANDI, E. ; TAMPIERI, A. ; CELOTTI, G. ; SPRIO, S.** Densification behaviour and mechanisms of synthetic hydroxyapatites. *J. Eur. Ceram. Soc.,* 2000, vol. 20, 2377-2387